# EUROPEAN PATENT SPECIFICATION

(11) **EP 2 275 566 B1**
(45) Date of publication and mention of the grant of the patent: **25.03.2015**
(21) Application number: 09275110.6
(22) Date of filing: 19.11.2009
(51) Int. Cl.: C12P 7/00, C12P 7/64, C12N 5/00

(54) **Method for cellular tissue multiplication from Jatropha curcas**
Verfahren zur Zellgewebevermehrung aus Jatropha curcas
Procédé pour la multiplication de tissus cellulaires extraits de Jatropha curcas

(30) Priority: 21.04.2009 CO 09040167
(43) Date of publication of application: 19.01.2011
(73) Proprietor: Empresas Públicas De Medellín E.S.P., Medellin, Antioquia (CO); Universidad De Antioquia, Medellin, Antioquia (CO)
(72) Inventor: Atehortua Garces, Lucia, Medellin, Antioquia (CO); Correa Cordoba, Sandra Marcela, Girardota, Antioquia (CO)
(74) Representative: Radkov, Stoyan Atanassov

(56) References cited:
- WO-A2-2009/133351
- WESELAKE R J ET AL: "TRIACYLGLYCEROL BIOSYNTHESIS AND GENE EXPRESSION IN MICROSPORE-DERIVED CELL SUSPENSION CULTURES OF OILSEED RAPE", JOURNAL OF EXPERIMENTAL BOTANY, OXFORD UNIVERSITY PRESS, GB LNKD- DOI:10.1093/JEXBOT/49.318.33, vol. 49, no. 318, 1 January 1998 (1998-01-01), pages 33-39, XP000881757, ISSN: 0022-0957
- WEN M-C ET AL: "COCOA BEAN CELL AND EMBRYO CULTURE", JOURNAL OF THE AMERICAN OIL CHEMISTS' SOCIETY, vol. 61, no. 11, 1984, pages 1720-1724, XP002602143, ISSN: 0003-021X
- ACHTEN W M J ET AL: "Jatropha bio-diesel production and use", BIOMASS AND BIOENERGY, vol. 32, no. 12, December 2008 (2008-12), pages 1063-1084, XP002602144, ISSN: 0961-9534
- JYOTI SARDANA ET AL: "An expeditious method for regeneration of somatic embryos in Jatropha curcas L", PHYTOMORPHOLOGY, INTERNATIONAL SOCIETY OF PLANT MORPHOLOGISTS, DELHI, IN, vol. 50, no. 3-4, 1 January 2000 (2000-01-01), pages 239-242, XP009144284, ISSN: 0031-9449
- MURASHIGE T ET AL: "A REVISED MEDIUM FOR RAPID GROWTH AND BIO ASSAYXS WITH TOBACCO TISSUE CULTURES", PHYSIOLOGIA PLANTARUM, MUNKSGAARD INTERNATIONAL PUBLISHERS, COPENHAGEN, DK, vol. 15, 1 January 1962 (1962-01-01), pages 473-497, XP000617351, ISSN: 0031-9317
- GAMBORG O L ET AL: "Plant tissue culture media", IN VITRO CELLULAR & DEVELOPMENT BIOLOGY. PLANT, GAITHERSBURG, MD, US, vol. 12, no. 7, 1 January 1976 (1976-01-01), pages 473-478, XP009146301, ISSN: 1054-5476

## Description

### BACKGROUND OF THE INVENTION

### 1. FIELD OF THE INVENTION

The present invention is related to the multiplication of cells derived from seeds of oleaginous plants and the obtention of oils derived from the generated cells.

### 2. DESCRIPTION OF PRIOR ART

The seeds of oleaginous plants had been identified as sources in the production of oils that could serve, among other uses, as biodiesel.

An example is the *Jatropha curcas* plant, which has a seed that contains oil. Said oil, in addition of having uses for medicine, veterinary, and soap-making, etc., is considered appropriate as a high quality biodiesel. The oil derived from the *Jatropha curcas* seed has physical-chemical and yield characteristics similar to diesel for engines (see Murali, et al., US Patent application Publication No. 2008/0194026 A1, paragraph 0008-0010).

Consequently, in the prior art, it has been described micro-propagation methods with the purpose of generating *Jatropha curcas* plants derived from organs, tissues, cells, or protoplasts. (see Murali, et al., paragraphs 0014,0017, and 0024). The generated plants could serve as source of seeds for the production of oil (see Shuyi Qiu, et al., China (CN) Patent Publication No. 11225416 A, Abstract).

Unfortunately, the possibility of oil production derived from the *Jatropha curcas* seed in big quantities for biodiesel use is limited because huge land areas are required for the harvest of plants generated by micro-propagation or conventional propagation.

The present invention provides a method for oil generation derived for *Jatropha curcas,* wherein plant growing is not required. Therefore, the method of the present invention does not need the use of land areas for oil generation derived from *Jatropha curcas,* or from other oleaginous plants.

### SUMMARY OF THE INVENTION

The present invention provides a method for the production of oil derived from multiplied cells from *Jatropha curcas* seed tissue, wherein the method is applicable to other oleaginous plant seeds. Said method comprises obtaining an explant from cotyledon from a *Jatropha curcas* seed, inoculating said explant in a culture medium with enzymes which break intercellular unions, in such a way that by incubating under agitation generates individual cells which are multiplied. From the multiplied cells derived from the individual cells derived from the *Jatropha curcas* seed cotyledon tissue, the oil that could be used a biodiesel is extracted.

Specifically, the method of the present invention comprises: Obtaining an explant from the *Jatropha curcas* seed; Putting the explant derived from the *Jatropha curcas* seed in a liquid culture medium; Breaking the intercellular unions of the tissue explants derived from the *Jatropha curcas* seed in said culture, wherein the explants generate individuals cells; Incubating for a determined time the culture medium with the individual cells generated from the explants derived from the *Jatropha curcas* seed, wherein said individual cells are multiplied within the determined time; and, Extracting oil from the cells that multiplied from the individual cells generated from the explants derived from the *Jatropha curcas* seed.

In one aspect of the method of the present invention, the explant is obtained from the *Jatropha curcas* seed by a procedure which comprises: Disinfecting the *Jatropha curcas* seed with ethanol, wherein said seed is bathed with ethanol; Hydrating said disinfected *Jatropha curcas* seed with a paper wet with sterile water, wherein the paper wet with sterile water is wrapped around the *Jatropha curcas* seed; Sterilizing the hydrated *Jatropha curcas* seed with sodium hypochlorite, wherein said hydrated *Jatropha curcas* seed is bathed with sodium hypochlorite; Retiring the skin of the sterilized *Jatropha curcas* seed, wherein as a result the seed cotyledon is released from the *Jatropha curcas* seed; and, Obtaining explants from the *Jatropha curcas* seed cotyledon.

The method of the present invention could be applied to any oleaginous plant. Said method adapted to any oleaginous plant comprises:
A. Obtaining an explant from the oleaginous plant seed;
B. Putting the explant derived from the seed in a culture medium;
C. Breaking the intercellular unions of the explants tissue derived from the seed in said culture medium, wherein the explants generate individual cells;
D. Incubating for a determined time the culture medium with the individual cells generated from the explants derived from the seed, wherein said individual cells are multiplied within the determined time; and,
E. Extracting oil from the cells that multiplied from the individual cells generated from the explants derived from the seed.

In another aspect of the method of the present invention, preferably, the culture medium contains at least NH₄NO₃, CaNO₃, CuSO₄, MnSO₄, ZnSO₄, H₃BO₃, KH₂PO₄. Na₂MoO₄, EDTA, FeSO₄, CaCl₂, CaCO₃, NaC₆H₇O₇ (sodium citrate), MgSO₄, K₂SO₄, thiamine, glycine, inositol, nicotinic acid, pyridoxine, biotin, glutamine, naftalenacetic acid, zeatin, and a carbon source.

In one aspect of the culture medium of the method of the present invention, said culture medium could contain a salt selected from the group that consist of CaNO₃, and KNO₃.

In one aspect of the culture medium of the method of the present invention, said culture medium could contain a salt selected from the group that consist of CaCl₂, and KCl.

In another aspect of the culture medium of the method of the present invention, said culture medium could contain an hormone selected from the group that consist of indolacetic acid (IAA), naftalenacetic acid (NAA), and indolebutyric acid (IBA).

In one aspect of the culture medium of the method of the present invention, said culture medium could contain an hormone selected from the group that consist of kinetine, benziladenine (BA), Gibberelline (GA), and Zeatin.

In one aspect of the culture medium of the method of the present invention, said culture medium could contain a carbon source selected from the group that consist of saccharose (sucrose), fructose, and glucose.

In another aspect of the culture medium of the method of the present invention, cellulase, pectinase and hemicellulase are added to the culture medium, wherein the cellulase, pectinase and hemicellulase break the tissue intercellular unions of the explants derived from the *Jatropha curcas* seed in said culture medium.

In one additional aspect of the method of the present invention, the oil is extracted from the culture medium with the individual cells that were multiplied from the individual cells generated from the explants derived from the *Jatropha curcas* seed by means of a procedure that comprises: Adding an organic solvent; Sonication; Centrifugation; Extraction of the superior face; and, Evaporation and drying of the solvent.

The present invention also provides a culture medium wherein the culture medium comprises NH₄NO₃, CaNO₃, CuSO₄, MnSO₄, ZnSO₄, H₃BO₃, KH₂PO₄, Na₂MoO₄, EDTA, FeSO₄, CaCl₂, CaCO₃, NaC₆H₇O₇ (sodium citrate), MgSO₄, K₂SO₄, thiamine, glycine, inositol, nicotinic acid, pyridoxine, biotin, glutamine, naftalenacetic acid, zeatin, and a carbon source.

In one aspect of the culture medium of the present invention, said culture medium contains cellulase, pectinase and hemicellulase, wherein the cellulase, pectinase and hemicellulase break the intercellular unions of the explants tissue derived from the *Jatropha curcas* seed in said culture medium.

In one aspect of the culture medium of the present invention, said culture medium could contain a salt selected from the group that consists of CaNO₃, and KNO₃.

In one aspect of the culture medium of the present invention, said culture medium could contain a salt selected from the group that consists of CaCl₂, and KCl.

In one aspect of the culture medium of the present invention, said culture medium could contain a hormone selected from the group that consists of indolacetic acid (IAA), naftalenacetic acid (NAA), and indolebutyric acid (IBA).

In one aspect of the culture medium of the present invention, said culture medium could contain a hormone selected from the group that consists of kinetine, benziladenine (BA), Gibberelline (GA), and Zeatin.

In one aspect of the culture medium of the present invention said culture medium could contain a carbon source selected from the group that consists of saccharose (sucrose), fructose, and glucose.

Additional objectives and advantages of the present invention will be more evident in the detailed description of the invention and the claims.

### DETAILED DESCRIPTION OF THE INVENTION

The method of the present invention comprises: Obtaining an explant from the *Jatropha curcas* seed; Putting the explant derived from the *Jatropha curcas* seed in a culture medium; Breaking the intercellular unions of the tissue explants derived from the *Jatropha curcas* seed in said culture medium, wherein the explants generate individuals cells; Incubating for a determined time the culture medium with the individual cells generated from the explants derived from the *Jatropha curcas* seed, wherein said individual cells are multiplied within the determined time; and, Extracting oil from the cells that multiplied from the individual cells generated from the explants derived from the *Jatropha curcas* seed.

In the preferred form of the method of the present invention, the preferred incubation time is at least 3 days from the moment in which the explant is put in the culture medium. In a more preferred form, the time of incubation is at least 8 days from the moment in which the explant is put in the culture medium. In a more preferred form yet the time of the incubation is at least 14 days from the moment in which the explant is put in the culture medium. The incubation is preferably performed under constant agitation at room temperature in darkness.

The incubation could also be done under illumination under a light source that emits a determined wave length, for example, infrared light, or a light source that emits the wave length that is optimal for multiplication of cells derived from a specific oleaginous plant seed.

The individual cells generated from the explants derived from the *Jatropha curcas* seed, once the intercellular unions had been broken after incubation of at least 3 days in the culture medium, can be used as a source of cells to re-inoculate in a fresh culture medium, repeating the re-inoculation repeatedly to fresh culture mediums thus enduring the multiplication of said individual cells. The preferred amount of individual cells that is re-inoculated to a fresh culture medium is from 1 to 2 x 10⁵ cells per milliliter, and said fresh re-inoculated culture medium is incubated for a determined time until the cells reach a multiplication stationary phase.

. In one aspect of the method of the present invention, the explant is obtained from the *Jatropha curcas* seed by a procedure which comprises: Disinfecting the *Jatropha curcas* seed with ethanol, wherein said seed is bathed with ethanol; Hydrating said disinfected *Jatropha curcas* seed with a paper wet with sterile water, wherein the paper wet with sterile water is wrapped around the *Jatropha curcas* seed; Sterilizing the hydrated *Jatropha curcas* seed with sodium hypochlorite, wherein said hydrated *Jatropha curcas* seed is bathed with sodium hypochlorite; Retiring the skin of the sterilized *Jatropha curcas* seed, wherein as a result the seed cotyledon is released from the *Jatropha curcas* seed; and, Obtaining explants from the *Jatropha curcas* seed cotyledon.

For purposes of the application of the present invention, the term explant refers to a portion of cotyledon from the *Jatropha curcas* seed. In the preferred form, the explant that is obtained from the *Jatropha curcas* seed cotyledon is constituted by transversally cut portions of cotyledon in laminar form from 1 to 2 mm of thickness. Explants could also be other kinds of cotyledon portions with different forms derived from the *Jatropha curcas* seed or seeds of other oleaginous plants. In the preferred form of the present invention the explant amount could be 1 to 7 grams per each 100 ml of culture medium.

The method of the present invention could be applied to any oleaginous plant. Said method adapted to any oleaginous plant comprises:
A. Obtaining an explant from the oleaginous plant seed;
B. Putting the explant derived from the seed in a culture medium;
C. Breaking the intercellular unions of the explants tissue derived from the seed in said culture medium, wherein the explants generate individual cells;
D. Incubating for a determined time the culture medium with the individual cells generated from the explants derived from the seed, wherein said individual cells are multiplied within the determined time; and,
E. Extracting oil from the cells that multiplied from the individual cells generated from the explants derived from the seed.

The term "oleaginous plant" is referred to any vegetable from which seed or fruit oil can be extracted, in some cases, edible, in other cases of industrial use and in other cases medicinal. Oleaginous plants include:
*Glycine max* (soy)
*Elaeis* guineensis (African Palm)
*Elaeis oleifera* (Noli or American oil palm)
*Arachis hypogaea* (Peanut)
*Helianthus annuus* (Sunflower)
Zea mays (corn)
*Linum usitatissimum* (Flax seeds)
*Brassica napus* (Colza, rape, canola or nabicol)
*Olea europaea L.* (Olive)
*Ricinus communis* (Castor oil)
*Sesamum indicum* (Sesame)
*Simmondsia chinensis* (Jojoba)
*Vernicia* fordii (Tung)
*Prunus dulcis* (Almond)
*Carthamus tinctorius* (safflower or alazor)
*Gossypium hirsutum* (cotton)
*Moringa oleifera* (drumstick tree)
*Triticum aestivum* (wheat)
and all classes of plants which seed has oil content.

In other aspect of the method of the present invention, preferably, the culture medium contains at least NH₄NO₃, CaNO₃, CuSO₄, MnSO₄, ZnSO₄, H₃BO₃, KH₂PO₄, Na₂MoO₄, EDTA, FeSO₄, CaCl₂, CaCO₃, NaC₆H₇O₇ (sodium citrate), MgSO₄, K₂SO₄, thiamine, glycine, inositol, nicotinic acid, pyridoxine, biotin, glutamine, naftalenacetic acid, zeatin, and a carbon source.

In one aspect of the culture medium of the method of the present invention, said culture medium could contain a salt selected from the group that consist of CaNO₃, and KNO₃.

In one aspect of the culture medium of the method of the present invention, said culture medium could contain a salt selected from the group that consist of CaCl₂, and KCl.

In one aspect of the culture medium of the method of the present invention, said culture medium could contain an hormone selected from the group that consist of indolacetic acid (IAA), naftalenacetic acid (NAA), and indolebutyric acid (IBA).

In one aspect of the culture medium of the method of the present invention, said culture medium could contain an hormone selected from the group that consist of kinetine, benziladenine (BA), Gibberelline (GA), and Zeatin.

In one aspect of the culture medium of the method of the present invention, said culture medium could contain a carbon source selected from the group that consist of saccharose (sucrose), fructose, and glucose.

In one aspect of the culture medium of the method of the present invention, cellulase, pectinase and hemicellulase are added to the culture medium, wherein the cellulase, pectinase and hemicellulase break the tissue intercellular unions of the explants derived from the *Jatropha curcas* seed in said culture medium. The intercellular unions of the explants tissue derived from the *Jatropha curcas* seed could be broken by others procedures enzymatic or non-enzymatic.

In the preferred form, the concentration ranges of the components of the culture medium of the present invention are the following:

| | |
|---|---|
| NH₄NO₃ | 100 - 500 mg/L |
| CaNO₃, or KNO₃ | 200 - 400 mg/L |
| CuSO₄ | 0.1 - 5 mg/L |
| MnSO₄ | 10 - 40 mg/L |
| ZnSO₄ | 20 - 50 mg/L |
| H₃BO₃ | 10 - 20 mg/L |
| KH₂PO₄ | 50 - 200 mg/L |
| Na₂MoO₄ | 0.1 - 5 mg/L |
| EDTA (ethylen-diamine-tetra-acetic acid) | 10 - 50 mg/L |
| FeSO₄ | 10 - 50 mg/L |
| CaCl₂, or KCl | 50 - 100 mg/L |
| CaCO₃ | 30 - 50 mg/L |
| NaC₆H₇O₇ (Sodium Citrate) | 0.1 - 10 mg/L |
| MgSO₄ | 50 - 300 mg/L |
| K₂SO₄ | 700 - 1500 mg/L |
| thiamine | 0.5 - 3 mg/L |
| glycine | 0.5 - 3 mg/L |
| inositol | 50 - 200 mg/L |
| nicotinic acid | 0.1 - 10 mg/L |
| pyridoxine | 0.1 - 10 mg/L |
| biotin | 0.1 - 10 mg/L |
| glutamine | 20 - 50 mg/L |
| IAA, or NAA, or IBA | 0.5 - 10 mg/L |
| zeatin, or kinetine, or BA, or GA | 0.5 - 10 mg/L |
| saccharose, or glucose, or fructose | 30000 - 100000 mg/L, and |
| hemicellulase, and pectinase, and cellulase | 3000 - 10000 mg/L |

In the preferred form of the method of the present invention the EDTA is disodic. The monosodic-EDTA can also be used in the method of the present invention.

In the preferred form of the method of the present invention the inositol is mio-inositol.

In the preferred form of the method of the present invention the culture medium is adjusted for a pH between 4.8 and 6.5.

In one additional aspect of the method of the present invention, the oil is extracted from the culture medium with the individual cells that were multiplied from the individual cells generated from the explants derived from the *Jatropha curcas* seed by means of a procedure that comprises: Adding an organic solvent; Sonication; Centrifugation; Extraction of the superior face; and, Evaporation and drying of the solvent.

In the case of *Jatropha curcas* or any other oleaginous plant, the oil extraction, from the cells that were multiplied from the individual cells generated from the explants derived from the seed cotyledon, is performed when the amount of cells reach an stationary phase in the culture medium. An organic solvent is added to the culture medium with the cells that were multiplied. The preferred organic solvent is hexane, thought other solvents like isopropanol or ethanol or others can be used.

The walls of the individual cells are broken when the mix of solvent, the culture medium and the individual cells are subjected to sonication, releasing the oil from the cells. The mix of solvent and culture medium that contains the broken cells and the released oil are centrifugated, which separates said mix in two phases, a superior one and an inferior one. The superior phase contains the solvent and the released oil, and the inferior phase contains said cells residues and the culture medium. The superior phase with the solvent and the oil is separated and then, the solvent of said phase is evaporated by roto-evaporation and heating, which results in the purification of oil.

The present invention also provides a culture medium wherein the culture medium comprises NH₄NO₃, CaNO₃, CuSO₄, MnSO₄, ZnSO₄, H₃BO₃, KH₂PO₄, Na₂MoO₄, EDTA, FeSO₄, CaCl₂, CaCO₃, NaC₆H₇O₇ (sodium citrate), MgSO₄, K₂SO₄, thiamine, glycine, inositol, nicotinic acid, pyridoxine, biotin, glutamine, naftalenacetic acid, zeatin, and a carbon source.

In one aspect of the culture medium of the present invention, said culture medium contains cellulase, pectinase and hemicellulase, wherein the cellulase, pectinase and hemicellulase break the intercellular unions of the explants tissue derived from the *Jatropha curcas* seed in said culture medium.

In one aspect of the culture medium of the method of the present invention, said culture medium could contain a salt selected from the group that consists of CaNO₃, and KNO₃.

In one aspect of the culture medium of the method of the present invention, said culture medium could contain a salt selected from the group that consists of CaCl₂, and KCl.

In one aspect of the culture medium of the present invention, said culture medium could contain a hormone selected from the group that consists of indolacetic acid (IAA), naftalenacetic acid (NAA), and indolebutyric acid (IBA).

In one aspect of the culture medium of the present invention, said culture medium could contain a hormone selected from the group that consists of kinetine, benziladenine (BA), Gibberelline (GA), and Zeatin.

In another aspect of the culture medium of the present invention, said culture medium could contain a carbon source selected from the group that consists of saccharose (sucrose), fructose, and glucose.

In the preferred form, the concentration ranges of the components of the culture medium of the present invention are the following:

| | |
|---|---|
| NH₄NO₃ | 100 - 500 mg/L |
| CaNO₃, or KNO₃ | 200 - 400 mg/L |
| CuSO₄ | 0.1 - 5 mg/L |
| MnSO₄ | 10 - 40 mg/L |
| ZnSO₄ | 20 - 50 mg/L |
| H₃BO₃ | 10 - 20 mg/L |
| KH₂PO₄ | 50 - 200 mg/L |
| Na₂MoO₄ | 0.1 - 5 mg/L |
| EDTA (ethylen-diamine-tetra-acetic acid) | 10 - 50 mg/L |
| FeSO₄ | 10 - 50 mg/L |
| CaCl₂, or KCl | 50 - 100 mg/L |
| CaCO₃ | 30 - 50 mg/L |
| NaC₆H₇O₇ (Sodium Citrate) | 0.1 - 10 mg/L |
| MgSO₄ | 50 - 300 mg/L |
| K₂SO₄ | 700 - 1500 mg/L |
| thiamine | 0.5 - 3 mg/L |
| glycine | 0.5 - 3 mg/L |
| inositol | 50 - 200 mg/L |
| nicotinic acid | 0.1 - 10 mg/L |
| pyridoxine | 0.1 - 10 mg/L |
| biotin | 0.1 - 10 mg/L |
| glutamine | 20 - 50 mg/L |
| IAA, or NAA, or IBA | 0.5 - 10 mg/L |
| zeatin, or kinetine, or BA, or GA | 0.5 - 10 mg/L |
| saccharose, or glucose, or fructose | 30000 - 100000 mg/L, and |
| hemicellulase, and pectinase, and cellulase | 3000 - 5000 mg/L |

In the preferred form of the culture medium of the present invention the EDTA is disodic. The monosodic-EDTA can also be used in the method of the present invention.

In the preferred form of the culture medium of the present invention the inositol is mio-inositol.

In the preferred form of the culture medium of the present invention is adjusted for a pH between 4.8 and 6.5.

Although the description presents preferred embodiments of the present invention, additional changes may be made in the form and disposition of the parts without deviating from the ideas and basic principles encompassed by the claims.

### EXAMPLES

Ripe *Jatropha curcas* seeds, previously cleaned and washed with water and soap, were disinfected with ethanol bathes; Then, said seeds were hydrated by been wrapped around with a paper wet with sterile water; subsequently, the seeds were sterilized with 3% sodium hypochlorite bathes; Then the skin of the seed was removed with a bistoury; From the resulting cotyledon of the *Jatropha curcas* seed explants or transversal laminar portions from 1 to 2 millimeters of thickness were cut.

Between 1 to 3 grams of explants of *Jatropha curcas* seed cotyledon were placed in 100 ml of culture medium with the following composition and the following concentrations:

| | |
|---|---|
| NH₄NO₃ | 400 mg/L |
| CaNO₃ | 383 mg/L |
| CuSO₄ | 0.25 mg/L |
| MnSO₄ | 22.3 mg/L |
| ZnSO₄ | 8.6 mg/L |
| H₃BO₃ | 6.2 mg/L |
| KH₂PO₄ | 170 mg/L |
| Na₂MoO₄ | 0.25 mg/L |
| Na₂EDTA (ethylen-diamine-tetra-acetic acid) | 37.3 mg/L |
| FeSO₄ | 27.85 mg/L |
| CaCl₂ | 72.5 mg/L |
| CaCO₃ | 20 mg/L |
| NaC₆H₇O₇ (Sodium Citrate) | 0.5 mg/L |
| MgSO₄ | 180.7 mg/L |
| K₂SO₄ | 990 mg/L |
| thiamine | 1 mg/L |
| glycine | 2 mg/L |
| mio-inositol | 100 mg/L |
| nicotinic acid | 0.5 mg/L |
| pyridoxine | 0.5 mg/L |
| biotin | 1 mg/L |
| glutamine | 30 mg/L |
| IAA | 2 mg/L |
| Kinetine | 1 mg/L |
| Saccharose | 50000 mg/L, and |
| pectinase, and cellulase | 5000 mg/L |

The culture medium was adjusted to a pH of 5.8 with HCL and/or NaOH 0.1 N solutions.

Said culture medium was incubated, under constant agitation, at room temperature, in the darkness for 15 days, when it was observed that explants tissue was converted to individually multiplied cells with a density of 2 x 10⁵ individual cells per milliliter approximately.

The cells were separated from the culture medium and were re-inoculated in 100 ml of fresh culture medium of the same composition and concentrations, continuing with the incubation under constant agitation, in the darkness, for approximately 10 more days, when the cells multiplying reached a stationary state (approximately 2 x 10⁶ individual cells per milliliter).

100 ml of hexane were added to the 100 ml of culture medium with individual cells in a density of approximately 2 x 10⁶ individual cells per milliliter. The resulting mix was subjected to sonication for 90 minutes. Then the mix was centrifugated at 2500 rpm for 30 minutes. A superior phase and an inferior phase resulted after centrifugation. The superior phase was separated and was transferred to a recipient which was roto-evaporated at 70°C, and at 180 rpm, and then it was placed in a stove for 1 hour at 75°C. The resulting oil weighted 1.978 grams (resulting yield of 19.78 grams/L).

The oil was analyzed with the results as shown in the following table:

**Table 1: Fatty acids estimated composition of oil sample.**

| Estimated composition of oil sample (gas chromatography acoplated to mass): | | |
|---|---|---|
| **FATTY ACID** | **RETENTION TIME (Min)** | **COMPOSITION %(p/p)** |
| Myristic | 11.766 | 0.26 |
| Nonanodioic acid | 13.155 | 0.15 |
| Palmitic acid | 13.460 | 19.96 |
| Palmitoleic | 13.698 | 0.61 |
| Estearic | 14.994 | 8.64 |
| Oleic | 15.188 | 38.46 |
| Linoleic | 15.555 | 31.35 |
| Eicosanoic | 16.387 | 0.56 |

The composition of the obtained oil from the individual cells derived from cotyledon of the *Jatropha curcas* seed, by the method of the present invention, is similar to the reported in literature, as it is shown in the following table extract from Akintayo, E:T:, Bioresource technology 92 (2004) 307-310.

**Table 3 of Akintayo, page 309.**

| Fatty acid composition (%)a of PKBS (*Parkia biglobbossa*) and JTC (*Jatropha curcas*)seeds oils | | |
|---|---|---|
| **Fatty acid** | **PKBS** | **JTC** |
| Palmitic | 27.5±0.5 | 19.5±0.8 |
| Estearic | 10.5±0.4 | 6.8±0.6 |
| Oleic | 14.5±0.5 | 41.3±1.5 |
| Linoleic | 44.5±1.5 | 31.4±1.2 |
| Linolenic | 3.0±0.2 - | |
| Saturated | 38 | 26.3 |
| Unsaturated | 62 | 72.7 |

| | | |
|---|---|---|
| Values are media ± standard deviation of duplicated determinations. | | |

The composition of oil derived from *Jatropha curcas* resulting from the method of the present invention is compared to the composition of the oil derived from *Jatropha curcas* described in literature. Therefore, the oil derived from *Jatropha curcas,* resulting from the method of the present invention, has the required characteristics for a high quality biodiesel as described in literature with respect to the oil derived from resulting from *Jatropha curcas.*

## Claims

1. A method for production of oil derived from multiplied individual cells from an explant of a plant seed wherein the method comprises:
a. obtaining an explant from the seed;
b. putting the explant derived from the seed in a culture medium;
c. breaking the intercellular unions of the explant tissue derived from the seed in said culture medium, wherein the explants generate individual cells;
d. incubating and multiplying for a determined time the individual cells generated from the explants derived from the plant seed in the culture medium; and,
e. extracting oil from the cells that multiplied from the individual cells generated from the explants derived from the plant seed.

2. A method for production of oil derived from multiplied individual cells from an explant of a *Jatropha curcas* seed wherein the method comprises:
a. obtaining an explant from the *Jatropha curcas* seed;
b. putting the explant derived from the *Jatropha curcas* seed in a culture medium;
c. breaking the intercellular unions of the explant tissue derived from the *Jatropha curcas* seed in said culture medium, wherein the explants generate individual cells;
d. incubating and multiplying for a determined time the individual cells generated from the explants derived from the plant seed in the culture medium; and,
e. extracting oil from the cells that multiplied from the individual cells generated from the explants derived from the *Jatropha curcas* seed.

3. The method of claims 1 or 2, wherein the explant is obtained from the plant seed by a procedure that comprises:
a. disinfecting the plant seed with ethanol, wherein said seed is bathed with ethanol;
b. hydrating said disinfected plant seed with a paper wet with sterile water, wherein the paper wet with sterile water is wrapped around the plant seed;
c. sterilizing the hydrated plant seed with sodium hypochlorite, wherein said hydrated plant seed is bathed with sodium hypochlorite;
d. removing the skin of the sterilized plant seed, wherein as a result the seed cotyledon is released from the plant seed; and,
e. obtaining explants from the plant seed cotyledon.

4. The method of any one of claims 1 to 3, wherein the incubation time from the moment in which the explant is put in the culture medium is at least 3 days.

5. The method of any one of claims 1 to 4, wherein the incubation time from the moment in which the explant is put in the culture medium is at least 8 days.

6. The method of any one of claims 1 to 5, wherein the incubation time from the moment in which the explant is put in the culture medium is at least 14 days.

7. The method of any one of claims 1 to 6 wherein the incubation time from the moment in which individual cells generated from the explants are re-inoculated in a fresh culture medium is at least 10 days.

8. The method of any one of claims 1 to 7, wherein the culture medium contains at least NH₄NO₃, CaNO₃, CuSO₄, MnSO₄, ZnSO₄, H₃BO₃, KH₂PO₄, Na₂MoO₄, EDTA, FeSO₄, CaCl₂, CaCO₃, NaC₆H₇O₇, MgSO4, K₂SO4, thiamine, glycine, inositol, nicotinic acid, pyridoxine, biotin, glutamine, naftalenacetic acid, zeatin, and a carbon source.

9. The method of any one of claim 1 to 8, wherein cellulase, pectinase and hemicellulase are added to the culture medium, wherein the cellulase, pectinase and hemicellulase break the intercellular unions of the explant tissue derived from the plant seed in said culture medium.

10. The method of any one of claim 1 to 9, wherein the culture medium contains a salt selected from the group that consists of CaNO₃ and KNO₃; and/or a salt selected from the group that consists of CaCl₂ and KCl.

11. The method of any one of claims 1 to 10, wherein the culture medium contains a hormone selected from the group that consists of indolacetic acid (IAA), naftalenacetic acid (NAA), and indolebutyric acid (IBA); and/or a hormone selected from the group that consists of kinetine, benziladenine (BA), Gibberelline (GA), and Zeatin.

12. The method of any one of claims 1 to 11, wherein the culture medium contains a carbon source selected from the group that consists of saccharose (sucrose), fructose, and glucose.

13. The method of any one of claims 1 to 12, wherein the oil is extracted from the culture medium with the individual cells that where multiplied from the individual cells generated from the explants derived from the plant seed by means of a procedure that comprises:
a. adding an organic solvent;
b. sonication;
c. centrifugation;
d. extraction of the superior phase; and,
e. evaporation and drying of the solvent.

## Patentansprüche

1. Verfahren zur Herstellung von Öl, das von vervielfachten Einzelzellen eines Explantats eines Pflanzensamens abgeleitet wird, wobei das Verfahren Folgendes umfasst:
a. Gewinnen eines Explantats aus dem Samen,
b. Geben des vom Samen abgeleiteten Explantats in ein Kulturmedium,
c. Aufbrechen der zwischenzellulären Verbindungen des vom Samen abgeleiteten Explantatgewebes im Kulturmedium, wobei die Explantate Einzelzellen erzeugen,
d. Inkubieren und Vervielfachen der Einzelzellen, die im Kulturmedium aus den vom Pflanzensamen abgeleiteten Explantaten erzeugt wurden, über einen bestimmten Zeitraum; und
e. Extrahieren von Öl aus den Zellen, die aus den Einzelzellen vervielfacht wurden, die aus den vom Pflanzensamen abgeleiteten Explantaten erzeugt wurden.

2. Verfahren zur Herstellung von Öl, das von vervielfachten Einzelzellen eines Explantats eines Jatropha-curcas-Samen abgeleitet wird, wobei das Verfahren Folgendes umfasst:
a. Gewinnen eines Explantats aus dem Jatropha-curcas-Samen,
b. Geben des vom Jatropha-curcas-Samen abgeleiteten Explantats in ein Kulturmedium,
c. Aufbrechen der zwischenzellulären Verbindungen des vom Jatropha-curcas-Samen abgeleiteten Explantatgewebes im Kulturmedium, wobei die Explantate Einzelzellen erzeugen,
d. Inkubieren und Vervielfachen der Einzelzellen, die im Kulturmedium aus den vom Pflanzensamen abgeleiteten Explantaten erzeugt wurden, über einen bestimmten Zeitraum; und
e. Extrahieren von Öl aus den Zellen, die aus den Einzelzellen vervielfacht wurden, die aus den vom Jatropha-curcas-Samen abgeleiteten Explantaten erzeugt wurden.

3. Verfahren nach Anspruch 1 oder 2, wobei das Explantat durch einen Vorgang aus dem Pflanzensamen gewonnen wird, der Folgendes umfasst:
a. Desinfizierend des Pflanzensamens mit Ethanol, wobei der Samen in Ethanol gebadet wird,
b. Hydratisieren des desinfizierten Pflanzensamens mit einem mit sterilem Wasser befeuchteten Papier, wobei das mit sterilem Wasser befeuchtete Papier um den Pflanzensamen gewickelt wird,
c. Sterilisieren des hydratisierten Pflanzensamens mit Natriumhypochlorit, wobei der hydratisierte Pflanzensamen in Natriumhypochlorit gebadet wird,
d. Entfernen der Haut des sterilisierten Pflanzensamens, wobei im Ergebnis das Keimblatt des Samens vom Pflanzensamen gelöst wird; und
e. Gewinnen von Explantaten aus dem Keimblatt des Pflanzensamens.

4. Verfahren nach einem der Ansprüche 1 bis 3, wobei die Inkubationszeit ab dem Zeitpunkt, an dem das Explantat in das Kulturmedium gegeben wird, mindestens 3 Tage beträgt.

5. Verfahren nach einem der Ansprüche 1 bis 4, wobei die Inkubationszeit ab dem Zeitpunkt, an dem das Explantat in das Kulturmedium gegeben wird, mindestens 8 Tage beträgt.

6. Verfahren nach einem der Ansprüche 1 bis 5, wobei die Inkubationszeit ab dem Zeitpunkt, an dem das Explantat in das Kulturmedium gegeben wird, mindestens 14 Tage beträgt.

7. Verfahren nach einem der Ansprüche 1 bis 6, wobei die Inkubationszeit ab dem Zeitpunkt, an dem Einzelzellen, die aus den Explantaten erzeugt wurden, erneut in ein frisches Kulturmedium geimpft werden, mindestens 10 Tage beträgt.

8. Verfahren nach einem der Ansprüche 1 bis 7, wobei das Kulturmedium mindestens NH₄NO₃, CaNO₃, CuSO₄, MnSO₄, ZnSO₄, H₃BO₃, KH₂PO₄, Na₂MoO₄, EDTA, FeSO₄, CaCl₂, CaCO₃, NaC₆H₇O₇, MgSO₄, K₂SO₄, Thiamin, Glycin, Inositol, Nikotinsäure, Pyridoxin, Biotin, Glutamin, Naphtalenessigsäure, Zeatin und eine Kohlenstoffquelle enthält.

9. Verfahren nach einem der Ansprüche 1 bis 8, wobei dem Kulturmedium Cellulase, Pektinase und Hemicellulase zugesetzt wird, wobei die Cellulase, Pektinase und Hemicellulase die zwischenzellulären Verbindungen des vom Pflanzensamen abgeleiteten Explantatgewebes im Kulturmedium aufbrechen.

10. Verfahren nach einem der Ansprüche 1 bis 9, wobei das Kulturmedium ein Salz enthält, das aus der Gruppe ausgewählt wird, die aus CaNO₃ und KNO₃ besteht, und/oder ein Salz das aus der Gruppe ausgewählt ist, die aus CaCl₂ und KCl besteht.

11. Verfahren nach einem der Ansprüche 1 bis 10, wobei das Kulturmedium ein Hormon enthält, das aus der Gruppe ausgewählt wird, die aus Indolessigsäure (IAA), Naphtalenessigsäure (NAA) und Indolbuttersäure (IBA) besteht, und/oder ein Hormon, das aus der Gruppe ausgewählt wird, die aus Kinetin, Benzyladenin (BA), Gibberellin (GA) und Zeatin besteht.

12. Verfahren nach einem der Ansprüche 1 bis 11, wobei das Kulturmedium eine Kohlenstoffquelle enthält, die aus der Gruppe ausgewählt wird, die aus Saccharose (Sucrose), Fructose und Glucose besteht.

13. Verfahren nach einem der Ansprüche 1 bis 12, wobei das Öl aus dem Kulturmedium mit den Einzelzellen, die aus den Einzelzellen vervielfacht wurden, die aus den vom Pflanzensamen abgeleiteten Explantaten erzeugt wurden, mittels eines Vorganges extrahiert wird, der Folgendes umfasst:
a. Zusetzen eines organischen Lösemittels,
b. Ultraschallbehandlung,
c. Zentrifugieren,
d. Extrahieren der übergeordneten Phase; und
e. Verdampfen und Eintrocknen des Lösemittels.

## Revendications

1. Procédé de production d'huile provenant de cellules individuelles multipliées à partir d'un explant d'une graine de plante, le procédé comprenant :
a. obtention d'un explant de la graine ;
b. introduction de l'explant provenant de la graine dans un milieu de culture ;
c. rupture des liaisons intercellulaires du tissu de l'explant provenant de la graine dans ledit milieu de culture, où les explants produisent des cellules individuelles ;
d. incubation et multiplication pendant un temps déterminé des cellules individuelles produites par les explants provenant de la graine de plante dans le milieu de culture ; et,
e. extraction d'huile des cellules qui se sont multipliées à partir des cellules individuelles produites par les explants provenant de la graine de plante.

2. Procédé de production d'huile provenant de cellules individuelles multipliées à partir d'un explant d'une graine de *Jatropha curcas*, le procédé comprenant :
a. obtention d'un explant à partir de la graine de *Jatropha curcas ;*
b. introduction de l'explant provenant de la graine de *Jatropha curcas* dans un milieu de culture ;
c. rupture des liaisons intercellulaires du tissu de l'explant provenant de la graine de *Jatropha curcas* dans ledit milieu de culture, où les explants produisent des cellules individuelles ;
d. incubation et multiplication pendant un temps déterminé des cellules individuelles produites par les explants provenant de la graine de plante dans le milieu de culture ; et,
e. extraction d'huile des cellules qui se sont multipliées à partir des cellules individuelles produites par les explants provenant de la graine de *Jatropha curcas.*

3. Procédé selon les revendications 1 ou 2, dans lequel l'explant est obtenu à partir de la graine de plante par un procédé qui comprend :
a. désinfection de la graine de plante au moyen d'éthanol, ladite graine étant baignée dans l'éthanol ;
b. hydratation de ladite graine de plante désinfectée au moyen d'un papier mouillé avec de l'eau stérile, le papier mouillé avec de l'eau stérile étant enroulé autour de la graine de plante ;
c. stérilisation de la graine de plante hydratée au moyen d'hypochlorite de sodium, ladite graine de plante hydratée étant baignée dans l'hypochlorite de sodium ;
d. élimination de la peau de la graine de plante stérilisée, le cotylédon de la graine étant en conséquence détaché de la graine de plante ; et,
e. obtention d'explants à partir du cotylédon de la graine de plante.

4. Procédé selon l'une quelconque des revendications 1 à 3, dans lequel le temps d'incubation à compter du moment où l'explant est introduit dans le milieu de culture est au moins de 3 jours.

5. Procédé selon l'une quelconque des revendications 1 à 4, dans lequel le temps d'incubation à compter du moment où l'explant est introduit dans le milieu de culture est au moins de 8 jours.

6. Procédé selon l'une quelconque des revendications 1 à 5, dans lequel le temps d'incubation à compter du moment où l'explant est introduit dans le milieu de culture est au moins de 14 jours.

7. Procédé selon l'une quelconque des revendications 1 à 6, dans lequel le temps d'incubation à compter du moment où les cellules individuelles produites par les explants sont réinoculées dans un milieu de culture frais est au moins de 10 jours.

8. Procédé selon l'une quelconque des revendications 1 à 7, dans lequel le milieu de culture contient au moins NH₄NO₃, CaNO₃, CuSO₄, MnSO₄, ZnSO₄, H₃BO₃, KH₂PO₄, Na₂MoO₄, EDTA, FeSO₄, CaCl₂, CaCO₃, NaC₆H₇O₇, MgS04, K₂SO₄, de la thiamine, de la glycine, de l'inositol, de l'acide nicotinique, de la pyridoxine, de la biotine, de la glutamine, de l'acide naphtalène acétique, de la zéatine et une source de carbone.

9. Procédé selon l'une quelconque des revendications 1 à 8, dans lequel de la cellulase, de la pectinase et de l'hémicellulase sont ajoutées au milieu de culture, où la cellulase, la pectinase et l'hémicellulase rompent les liaisons intercellulaires du tissu de l'explant provenant de la graine de plante dans ledit milieu de culture.

10. Procédé selon l'une quelconque des revendications 1 à 9, dans lequel le milieu de culture contient un sel choisi dans le groupe constitué de CaNO₃ et KNO₃ ; et/ou un sel choisi dans le groupe constitué de CaCl₂ et KCl.

11. Procédé selon l'une quelconque des revendications 1 à 10, dans lequel le milieu de culture contient une hormone choisie dans le groupe constitué d'acide indole acétique (IAA), d'acide naphtalène acétique (NAA) et d'acide indole butyrique (IBA) ; et/ou une hormone choisie dans le groupe constitué de kinétine, de benziladénine (BA), de gibbérelline (GA) et de zéatine.

12. Procédé selon l'une quelconque des revendications 1 à 11, dans lequel le milieu de culture contient une source de carbone choisie dans le groupe constitué de saccharose (sucrose), de fructose et de glucose.

13. Procédé selon l'une quelconque des revendications 1 à 12, dans lequel l'huile est extraite du milieu de culture avec les cellules individuelles qui se sont multipliées à partir des cellules individuelles produites par les explants provenant de la graine de plante au moyen d'un procédé qui comprend :
a. ajout d'un solvant organique ;
b. sonication ;
c. centrifugation ;
d. extraction de la phase supérieure ; et,
e. évaporation et séchage du solvant.
